(19) Europäisches Patentamt
European Patent Office
Office européen des brevets

(11) Veröffentlichungsnummer: **0 056 966**
**B1**

(12) **EUROPÄISCHE PATENTSCHRIFT**

(45) Veröffentlichungstag der Patentschrift: **10.10.84**

(51) Int. Cl.³: **C 07 D 263/44** // C07C103/58, C07C103/375

(21) Anmeldenummer: **82100306.8**

(22) Anmeldetag: **18.01.82**

(54) **Verfahren zur Herstellung von Oxazolidin-2,4-dionen.**

(30) Priorität: **24.01.81 DE 3102359**

(43) Veröffentlichungstag der Anmeldung: **04.08.82 Patentblatt 82/31**

(45) Bekanntmachung des Hinweises auf die Patenterteilung: **10.10.84 Patentblatt 84/41**

(84) Benannte Vertragsstaaten: **AT BE CH DE FR GB IT LI LU NL SE**

(56) Entgegenhaltungen:
**EP-A-0 004 582**
**EP-A-0 007 415**
**DE-A-1 813 434**
**FR-A-2 316 239**
**US-A-2 338 220**
**US-A-2 909 467**
**US-A-2 928 840**

(73) Patentinhaber: **BASF Aktiengesellschaft, Carl-Bosch-Strasse 38, D-6700 Ludwigshafen (DE)**

(72) Erfinder: **Scholz, Herbert, Dr., Im Finkenschlag 16, D-6730 Neustadt (DE)**

**EP 0 056 966 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein neues Verfahren zur Herstellung von Oxazolidin-2,4-dionen durch Umsetzung von substituierten Amiden mit aromatischen Kohlensäureestern.

Es ist bekannt, Oxazolidin-2,4-dione durch Umsetzung von Isocyanaten mit 2-Hydroxycarbonsäureestern herzustellen (DE-AS 1 811 843, DE-OS 2 022 494, DE-OS 2 207 576).

Es ist ferner bekannt, ein substituiertes Harnstoffderivat mit Diphenylcarbonat zu einer Verbindung mit einem 6-Ring umzusetzen, die 3 Stickstoffatome im Ring enthält (FR-A-2 316 239).

Es ist ferner bekannt, ein primäres Hydroxycarbonsäureamid mit Dialkylcarbonat und einer stöchiometrischen Menge eines Metallalkoholats in 2 Stufen zu einem Oxazolidindion umzusetzen (US-A-2 338 220 und US-A-2 909 467).

Durch die bekannten Verfahren wird das neue Verfahren nicht nahegelegt, weil in den bekannten Verfahren entweder völlig andere Ausgangs- und Endprodukte auftreten oder weil das Verfahren in wesentlich anderer Weise durchgeführt wird.

Es wurde nun gefunden, daß man ein Oxazolidin-2,4-dion der Formel

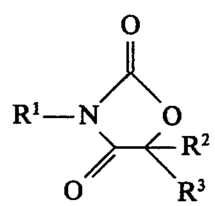

in der

- $R^1$ einen Naphthylrest oder einen gegebenenfalls durch ein oder mehrere Halogenatome, Methyl oder Methoxy substituierten Phenylrest,
- $R^2$ Wasserstoff oder einen Alkylrest mit 1 bis 4 C-Atomen, Halogen oder einen Vinylrest oder Methoxymethyl oder Carboäthoxy bedeutet und
- $R^3$ die gleichen Bedeutungen wie $R^2$ hat, wobei $R^2$ und $R^3$ im Einzelfall gleich oder verschieden sind oder $R^2$ und $R^3$ miteinander verbunden sind und je einen Methylenrest bedeuten,

in sehr guter Ausbeute erhält, wenn man ein Amid der Formel

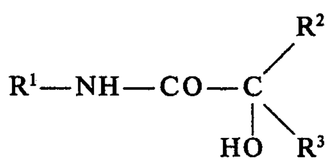

in der $R^1$, $R^2$ und $R^3$ die oben genannten Bedeutungen haben bei einer Temperatur zwischen 50 und 250° C mit einem aromatischen Kohlensäureester der Formel

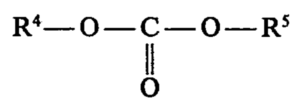

umsetzt, in der

- $R^4$ einen gegebenenfalls durch Methyl oder Halogen substituierten Phenylrest bedeutet und
- $R^5$ die gleiche Bedeutung wie $R^4$ hat oder $R^4$ und $R^5$ zusammen den 1,2-Phenylen-Rest bedeuten,

und die bei der Umsetzung entstehenden Phenole $R^4OH$ und $R^5OH$ abtrennt, vorzugsweise abdestilliert.

Die verschiedenen Substituenten haben beispielsweise die folgenden Bedeutungen:

| | |
|---|---|
| $R^1$ | ist z. B. Phenyl, Naphtyl, 2-, 3- und 4-Fluorphenyl, 2-, 3- und 4-Chorphenyl, 2-, 3- und 4-Bromphenyl, 2,3-Dichlorphenyl, 2,3,6-Trichlorphenyl, 2-, 3- und 4-Methoxyphenyl, 2-, 3- und 4-Methylphenyl, 3,4-Dichlorphenyl, 2,6-Dichlorphenyl, 2,3,4-Trichlorphenyl, 2,3,5-Trichlorphenyl und bevorzugt 3,5-Dichlorphenyl. |
| $R^2$ und $R^3$ | sind z. B. Wasserstoff oder Vinyl oder Alkyl, z. B. Methyl, Äthyl oder Methoxymethyl oder Carboäthoxy. |
| $R^4$ und $R^5$ | bedeuten z. B. 4-Methylphenyl, 4-Chlorphenyl oder bevorzugt Phenyl oder $R^4$ und $R^5$ zusammen 1,2-Phenylen |

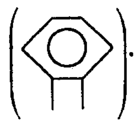

Für die Durchführung der Umsetzung ist eine erhöhte Temperatur zwischen 50 und 250° C, vorzugsweise zwischen 120 und 170° C erforderlich.

Für die Reaktion ist wichtig, daß der aromatische Kohlensäureester weitgehend rein ist (z. B. frei von Chlorameisensäurephenylester und sauren Verunreinigungen).

Die Reaktion kann ohne Katalysator durchgeführt werden. Um aber möglichst quantitative und schnelle Umsetzungen, d.h. hohe Raum-Zeit-Ausbeuten zu erhalten, kann der Zusatz von folgenden basischen Katalysatoren von Vorteil sein: Alkalialkoholate wie Natriummethylat, Natriumäthylat, Amine z. B. tertiäre Amine (Tripropylamin, Tributylamin), Alkaliphenolate der Formel

$$R^4O^{\ominus}Me^+$$

wobei $R^4$ die obige Bedeutung hat und Me ein Alkalimetall bedeutet (z. B. Natriumphenolat). Der Zusatz der Katalysatoren erfolgt z. B. in einer Menge von 0,01 Molprozent bis 0,1 Molprozent, bezogen auf das Amid.

Die Umsetzung wird vorteilhaft ohne Lösungsmittel durchgeführt.

Falls Löungsmittel verwendet werden, sollte deren Siedepunkt über dem der Phenole $R^4OH$ und $R^5OH$ liegen.

Es ist überraschend, daß die Umsetzung mit dem Diarylcarbonat sehr rasch und ohne Katalysator erfolgt.

Wichtig für eine gute Ausbeute bei der Umsetzung ist es, die bei der Umsetzung entstehenden Phenole $R^4OH$ und $R^5OH$ möglichst quantitativ abzutrennen, vorzugsweise abzudestillieren und dafür zu sorgen, daß das als Ausgangsprodukt verwendete Diarylcarbonat nicht mitdestilliert. Zu diesem Zweck ist die Verwendung von Destillationskolonnen mit Füllkörpern besonders vorteilhaft.

Die abdestillierten Phenole $R^4OH$ und $R^5OH$ fallen so rein an, daß sie direkt als Einsatzstoffe für die Herstellung von

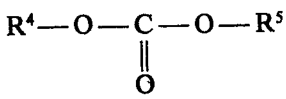

wieder verwendet werden können (recycling).

Die Umsetzung kann bei Normaldruck durchgeführt werden; ein Vakuum von ca. 1 mbar bis 950 mbar kann von Vorteil sein, um die Destillationstemperatur zu erniedrigen.

Die als Ausgangsprodukte verwendeten Amide entstehen z. B. als Nebenprodukte bei der bekannten Herstellung der entsprechenden Oxazolidin-2,4-dione.

Die Amide entstehen auch bei der Alkoholyse der entsprechenden Oxazolidin-2,4-dione mit Äthylenglykol oder Glyzerin.

Das Verfahren kann allgemein formuliert werden:

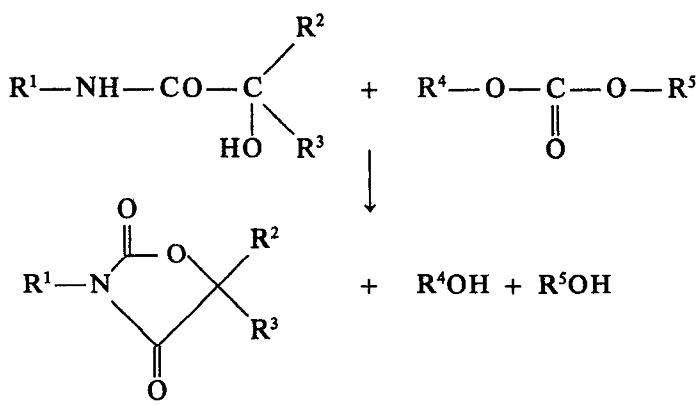

Der billigere Reaktionspartner z. B.

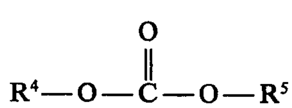

kann im Überschuß (z. B. 1,1 Mol Ester zu 1 Mol Amid) eingesetzt werden.

Die folgenden Beispiele erläutern die Durchführung des erfindungsgemäßen Verfahrens:

Beispiel 1

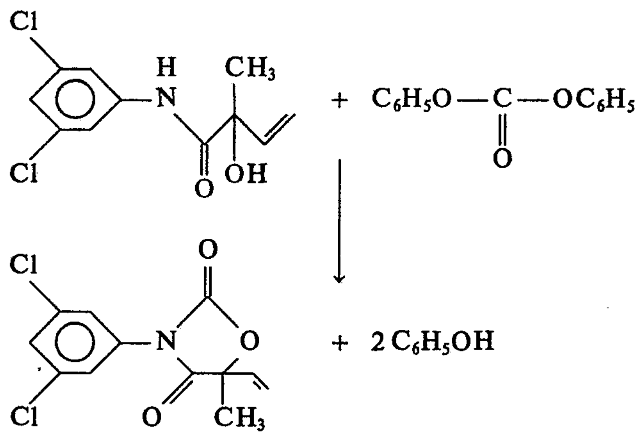

26 g (0,1 mol) N-(3,5-Dichlorphenyl)-2-hydroxy-2-methyl-3-butensäureamid und 21,4 g (0,1 mol) Diphenylcarbonat werden auf 160—165° C erhitzt. Bei einem Druck von ca. 30 mbar destillieren ca. 18,4 g Phenol (Übergangstemperatur 88—95° C) über. Die verbleibende Schmelze wird auf ca. 80° C abgekühlt und es werden 25 ml Methanol zugesetzt. Man rührt 2 Stunden und kühlt auf 5° C ab. Nach dem Absaugen wäscht man zweimal mit je 10 ml Methanol und anschließend mit 50 ml Wasser.

Ausbeute: 26,7 g 3-(3,5-Dichlorphenyl)-5-methyl-5-vinyl-1,3-oxazolidin-2,4,dion, Gehalt 99% = 92,4% Ausbeute ber. für 100%iges Produkt. Fp 111° C, dünnschichtchromatographisch sauber.

Beispiel 2

N-(3,5-Dichlorphenyl)-5-methyl-5-methoxymethyl-oxazolidin-2,4-dion

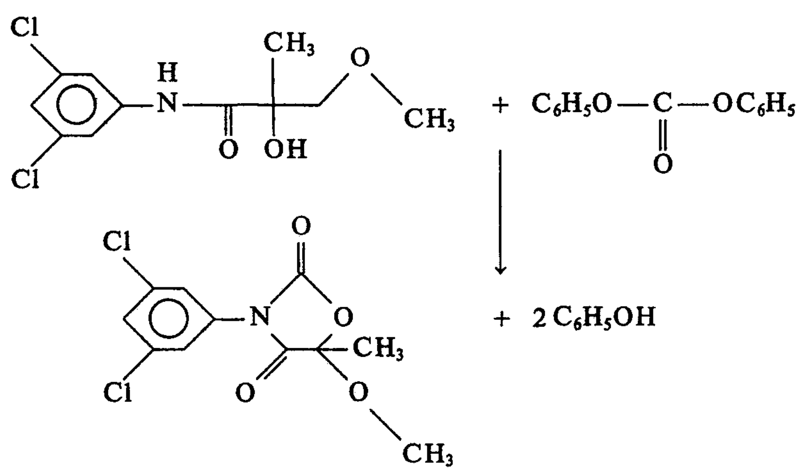

27,8 g (0,1 mol) N-(3,5-Dichlorphenyl)-2-hydroxy-2-methoxymethyl-propansäureamid, 21,4 g (0,1 mol) Diphenylcarbonat und 0,7 g (0,01 mol) Natriumäthylat werden auf 140—145° C erhitzt. Bei 22 mbar wird in 1 Stunde das entstehende Phenol (Übergang 88—102° C) abdestilliert. Der Rückstand wird auf 70° C abgekühlt und mit 75 ml Methanol versetzt und 2 Stunden bei 15° C gerührt. Nach dem Absaugen wird zweimal mit je 10 ml Wasser gewaschen und getrocknet.

Ausbeute: 13 g N-(3,5-Dichlorphenyl)-5-methyl-5-methoxy-methyl-oxazolidin-2,4-dion, das entspricht 85,5% Ausbeute.

Fp: 112° C (dünnschichtchromatographisch sauber).

Gehalt: 100% (bestimmt durch Hochdruckflüssigkeitschromatographie). Die folgenden Vorschriften erläutern die Herstellung der Amide durch Alkoholyse.

Vorschrift 1

N-(3,5-Dichlorphenyl)-2-hydroxy-2-methyl-3-butensäureamid

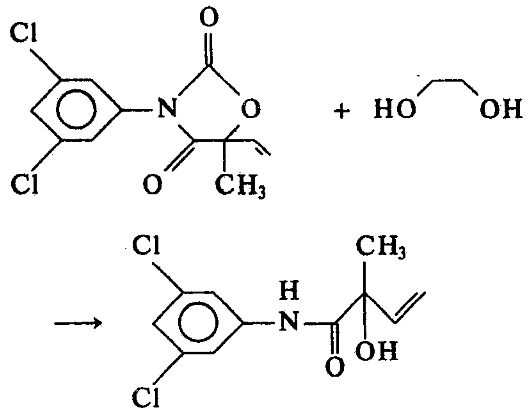

214,5 g (0,75 mol) 3-(3,5-Dichlorphenyl)-5-methyl-5-vinyl-1,3-oxazolidin-2,4-dion werden mit 750 ml (13,5 mol) Äthylenglykol und 8,25 g (0,056 mol) Tripropylamin 2 Stunden bei 150° C gerührt.

Bei ca. 80° C wird anschließend am Rotationsverdampfer mit der Vakuumpumpe (ca. 0,2 mbar) das restliche Äthylenglykol abdestilliert. Der Rückstand, der beim Stehen über Nacht auskristallisiert, wiegt 198 g und kann aus 150 ml Toluol unkristallisiert werden. Man erhält 128 g N-(3,5-Dichlorphenyl)-2-hydroxy-2-methyl-3-butensäureamid.

Fp 116° C. IR- und H-NMR-Spektren stimmen mit der Struktur überein.

Analyse

| | |
|---|---|
| gef.: | C 50,9, H 4,4, O 12,3, N 5,9, Cl 27,2, |
| ber.: | C 50,8, H 4,2, O 12,3, N 5,4, Cl 27,3. |

Vorschrift 2

N-(3,5-Dichlorphenyl)-2-hydroxy-2-methoxymethyl-propansäureamid

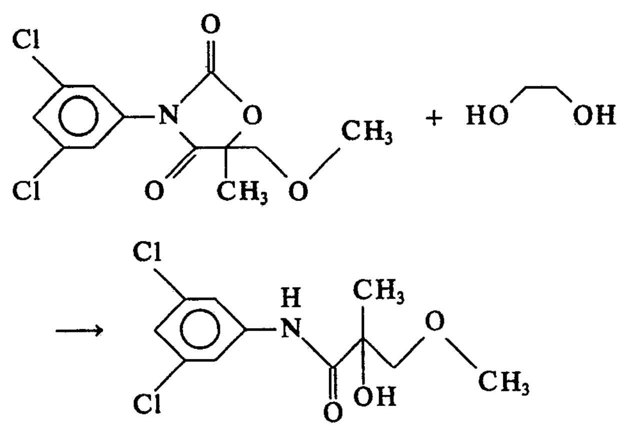

30,4 g (0,1 mol) N-(3,5-Dichlorphenyl)-5-methyl-5-methoxy-methyl-oxazolidin-2,4-dion und 100 ml (1,8 mol) Äthylenglykol, sowie 1,1 g (0,0075 mol) Tripropylamin werden 2 Stunden bei 150° C gerührt. Aufarbeitung wie in Vorschrift 1 beschrieben. Umkristallisieren aus Toluol.

Ausbeute: 22 g = 79,1% N-(3,5-Dichlorphenyl)-2-hydroxy-2-methoxymethylpropansäureamid. Fp: 140° C.

**Patentansprüche**

1. Verfahren zur Herstellung von Oxazolidin-2,4-dionen der Formel

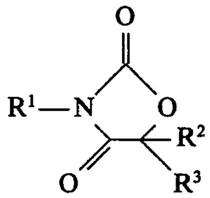

in der

- $R^1$ einen Naphthylrest oder einen gegebenenfalls durch ein oder mehrere Halogenatome, Methyl oder Methoxy substituierten Phenylrest,
- $R^2$ Wasserstoff oder einen Alkylrest mit 1 bis 4 C-Atomen, Halogen oder einen Vinylrest oder Methoxymethyl oder Carboäthoxy bedeutet und
- $R^3$ die gleichen Bedeutungen wie $R^2$ hat, wobei $R^2$ und $R^3$ im Einzelfall gleich oder verschieden sind oder $R^2$ und $R^3$ miteinander verbunden sind und je einen Methylenrest bedeuten,

durch Umsetzung eines Hydroxycarbonsäureamids mit einem Diorganylcarbonat, dadurch gekennzeichnet, daß man ein Amid der Formel

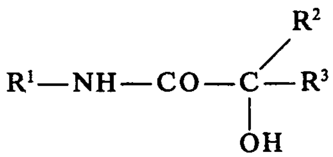

in der $R^1$, $R^2$ und $R^3$ die oben genannten Bedeutungen haben, bei einer Temperatur zwischen 50 und 250° C mit einem Kohlensäureester der Formel

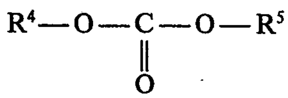

umsetzt, in der

- $R^4$ einen gegebenenfalls durch Methyl oder Halogen substituierten Phenylrest bedeutet und
- $R^5$ die gleiche Bedeutung wie $R^4$ hat oder $R^4$ und $R^5$ zusammen den 1,2-Phenylen-Rest bedeuten,

und die bei der Umsetzung entstehenden Phenole $R^4OH$ und $R^5OH$ abtrennt, vorzugsweise abdestilliert.

2. Verfahren gemäß Anspruch 1, dadurch gekennzeichnet, daß man die Umsetzungen mit Verbindungen durchführt, bei denen $R^1$ 3,5-Dichlorphenyl, $R^2$ Methyl und $R^3$ Vinyl bedeuten.

3. Verfahren gemäß Anspruch 1, dadurch gekennzeichnet, daß man die Umsetzungen mit Verbindungen durchführt, bei denen $R^1$ 3,5-Dichlorphenyl, $R^2$ Methyl und $R^3$ Methoxymethyl bedeuten.

**Claims**

1. A process for the preparation of oxazolidine-2,4-diones or the formula

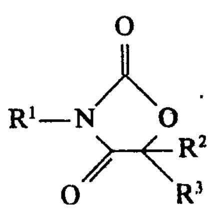

where

- $R^1$ is naphthyl, or phenyl which is unsubstituted or substituted by one or more halogen atoms, methyl or methoxy,

$R^2$ is hydrogen, alkyl of 1 to 4 carbon atoms, halogen, vinyl, methoxymethyl or carboethoxy, and

$R^3$ has the same meanings as $R^2$, $R^2$ and $R^3$ being identical or different, or $R^2$ and $R^3$ are bonded to one another and each is methylene,

by reacting a hydroxycarboxylic acid amide with a diorganyl carbonate, wherein an amide of the formula

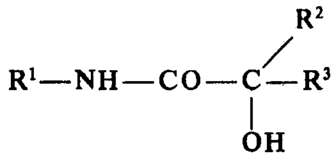

where $R^1$, $R^2$ and $R^3$ have the above meanings, is reacted with a carbonic acid ester of the formula

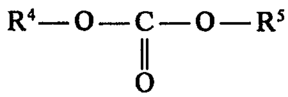

where

$R^4$ is phenyl which is unsubstituted or substituted by methyl or halogen and

$R^5$ has the same meaning as $R^4$, or $R^4$ and $R^5$ together are 1,2-phenylene,

at from 50 to 250°C, and the phenols $R^4OH$ and $R^5OH$ formed during the reaction are isolated, preferably by distillation.

2. A process as claimed in claim 1, wherein the reaction is carried out with a compound where $R^1$ is 3,5-dichlorophenyl, $R^2$ is methyl and $R^3$ is vinyl.

3. A process as claimed in claim 1, wherein the reaction is carried out with a compound where $R^1$ is 3,5-dichlorophenyl, $R^2$ is methyl and $R^3$ is methoxymethyl.

**Revendications**

1. Procédé de préparation d'oxazolidine-2,4-diones de la formule

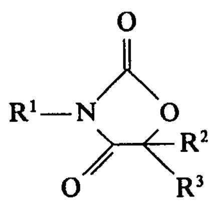

dans laquelle

$R^1$ désigne un groupe naphtyle ou un groupe phényle éventuellement substitué par un ou plusieurs atomes d'halogène ou radicaux méthyle ou méthoxy,

$R^2$ désigne un atome d'hydrogène ou d'halogène, un radical alkyle en $C_1$ à $C_4$ ou un groupe vinyle, méthoxy-méthyle ou carbéthoxy et

$R^3$ possède les mêmes significations que $R^2$,$R^2$ et $R^3$ pouvant être identiques ou différents, $R^2$ et $R^3$ pouvant aussi représenter deux groupes méthylène liés l'un à l'autre,

par réaction d'un amide d'acide hydroxy-carboxylique avec un diorganyl-carbonate, caractérisé en ce que l'on fait réagir un amide de la formule

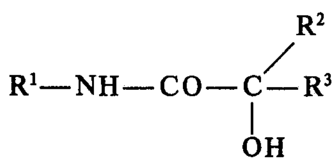

dans laquelle $R^1$, $R^2$ et $R^3$ possèdent les significations définies, à une température comprise entre 50 et 250° C, avec un ester d'acide carbonique de la formule

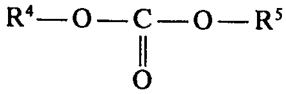

dans laquelle

$R^4$ désigne un groupe phényle éventuellement halo- ou méthyl-substitué et

$R^5$ possède la même signification que $R^4$ ou $R^4$ et $R^5$ forment ensemble un groupe 1,2-phénylène,

avec séparation, de préférence par distillation, des phénols $R^4OH$ et $R^5OH$ formés pendant la réaction.

2. Procédé suivant la revendication 1, caractérisé in ce que les réactions sont réalisées avec des composés, pour lesquels $R^1$ = dichloro-3,5 phényle, $R^2$ = méthyle et $R^3$ = vinyle.

3. Procédé suivant la revendication 1, caractérisé en ce que les réactions sont réalisées avec des composés, pour lesquels $R^1$ = dichloro-3,5 phényle, $R^2$ = méthyle et $R^3$ = méthoxy-méthyle.